(19) Europäisches Patentamt
European Patent Office
Office européen des brevets

(11) Veröffentlichungsnummer: **0 003 141**
**B1**

(12) **EUROPÄISCHE PATENTSCHRIFT**

(45) Veröffentlichungstag der Patentschrift: **14.07.82**

(51) Int. Cl.³: **C 07 D 277/82**, C 07 D 277/84, C 07 D 277/60

(21) Anmeldenummer: **79100117.5**

(22) Anmeldetag: **16.01.79**

(54) **Verfahren zur Herstellung von 2-Amino-arylenothiazol-Verbindungen und von deren im Ring N-substituierten Derivaten.**

(30) Priorität: **18.01.78 DE 2801991**
**09.08.78 DE 2834852**
**18.10.78 DE 2845250**

(43) Veröffentlichungstag der Anmeldung:
**25.07.79 Patentblatt 79/15**

(45) Bekanntmachung des Hinweises auf die Patenterteilung:
**14.07.82 Patentblatt 82/28**

(84) Benannte Vertragsstaaten:
**BE CH DE FR GB IT**

(56) Entgegenhaltungen:
**FR-A-2 298 548**
**GB-A-345 735**
**JOURNAL OF INDIAN CHEMICAL SOCIETY, 1 Vol. 44 (11), 1967, Calcutta,**
**P. H. DESHPANDE: »Ocisdation of 1-tert butyl-3-phenylthiocarbamide with bromine and thionyl chloride«, Seiten 951—954**

(73) Patentinhaber: **HOECHST AKTIENGESELLSCHAFT, Postfach 80 03 20, D-6230 Frankfurt/Main 80 (DE)**

(72) Erfinder: **Papenfuhs, Theodor, Dr., Heinrich-Bleicher-Strasse 40, D-6000 Frankfurt/Main 50 (DE)**

**EP 0 003 141 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von 2-Amino-arylenothiazol-Verbindungen und von deren im Ring N-substituierten Derivaten

Die vorliegende Erfindung liegt in einer Verfahrensverbesserung zur Herstellung von Zwischenprodukten, die beispielsweise als Ausgangsstoffe, insbesondere für Azofarbstoffe, verwendet werden; sie betrifft ein verbessertes Verfahren zur Herstellung von 2-Amino-arylenothiazolen und deren im Heterocyclus N-substituierten Derivaten, d. h. von 2-Imino-arylenothiazolinen, durch Cyclisierung von N-Aryl-thioharnstoffen, von N'-substituierten N-Aryl-thioharnstoffen bzw. von N-substituierten N-Aryl-thioharnstoffen.

2-Aminoarylenothiazole, wie 2-Aminobenzothiazole und 2-Amino-naphthothiazole, deren 2-N-substituierte Derivate sowie am Ring-Stickstoff substituierte 2-Imino-arylenothiazoline sind wertvolle Ausgangskomponenten für Azofarbstoffe und Pflanzenschutzmittel. Sie können durch oxidativen Ringschluß von N-Aryl-thioharnstoffen, N'-substituierten N-Arylthioharnstoffen bzw. N-substituierten N-Aryl-thioharnstoffen hergestellt werden. Als Cyclisierungsmittel wurden dafür schon Brom und Bromchlorid (BrCl), Brom in Chloroform oder Essigsäure (Journal für praktische Chemie [2] 153, 1 (1939); Org. Synthesis 27, 53 (1947); J. Chem. Soc. 1926, 2951 u. 2958; J. Chem. Soc. 1927, 1209) sowie Chlor in Äthylendichlorid mit Brom als Katalysator (Deutsche Offenlegungsschrift 2 631 163), des weiteren Dischwefeldichlorid ($S_2Cl_2$), Sulfurylchlorid ($SO_2Cl_2$) und Schwefeldichlorid ($SCl_2$) vorgeschlagen, wobei technisch vorwiegend Dischwefeldichlorid verwendet wird (vgl. BIOS 1149, S. 83; J. Chem. Soc. London, (C), 1969, S. 268; DE-OS 1 916 599; Elderfield, Heterocyclic Compounds, Vol. 5, S. 581 ff. (1957); FR-PS 688 867). Bei diesen Verfahren wird in organischen Lösungsmitteln gearbeitet, was bei technischer Durchführung jedoch gern vermieden wird. Zwar ist aus der DE-OS 2 601 700 auch eine lösungsmittelfreie Cyclisierungsvariante bekanntgeworden; diese hat jedoch durch Verwendung eines mindestens 10fachen Überschusses an Dischwefelchlorid keinerlei technisches Interesse erlangt. Nachteilig ist aber insbesondere bei den bekannten technisch durchgeführten Verfahren bei Verwendung von Dischwefeldichlorid die Bildung von Harzen, der hohe Anfall an Schwefel, der etwa 7 Mol pro Mol 2-Amino-arylenothiazol bzw. 2-Imino-arylenothioazolin beträgt, sowie zusätzlich die zeitaufwendige Regeneration des Lösungsmittels bzw. der hohe Zeitbedarf für die Wasserdampfdestillation.

Aus der britischen Patentschrift 345 735 ist die Verwendung von Sulfurylchlorid als Cyclisierungsmittel zur Herstellung von am Stickstoff gegebenenfalls substituierten 2-Amino-arylenothiazolen oder deren im Thiazolinring N-substituierten 2-Imino-arylenothiazoline aus den entsprechenden Arylthioharnstoffen bekannt. Sulfurylchlorid hat jedoch den erheblichen Nachteil, daß es zugleich ein chlorabgebendes, d. h. chlorierendes, Mittel ist, so daß mit dem Ringschluß der Arylthioharnstoffe zu den 2-Amino-arylenothiazol-Derivaten gleichzeitig eine teilweise Chlorierung der Arylkerne erfolgt.

Hingegen besitzt Thionylchlorid diese kernchlorierende Eigenschaft nicht. Seine Verwendung als Oxidationsmittel als Analoges zu Brom wird in Journ. Indian Chem. Soc. 44, 951 – 954 (1967), zur Oxidation von N'-tert.Butyl-N-phenylthioharnstoff beschrieben; hierbei erfolgt unter Dimerisierung und teilweiser Entschwefelung Cyclisierung zu einem kompliziert aufgebauten 2-Aminobenzothiazol-Derivat, jedoch bildet sich nicht das bei einfachem Ringschluß zu erwartende 2-N-(tert.-Butyl)-amino-benzothiazol. In Säure zerfällt dieses dimere Produkt vollständig unter teilweiser Bildung von unsubstituiertem 2-Amino-benzothiazol.

Mit der vorliegenden Erfindung wurden die vorstehend beschriebenen Nachteile behoben. Sie betrifft ein verbessertes und wesentlich einfacher durchzuführendes Verfahren zur Herstellung von in der 2-ständigen Aminogruppe unsubstituierten 2-Amino-arylenothiazolen und deren im Thiazolinring durch Alkyl-, Cycloalkyl- oder Arylreste N-substituierten Verbindungen, d. h. deren 2-Imino-arylenothiazolinen, sowie von 2-Amino-arylenothiazolen, deren in 2-Stellung befindliche Aminogruppe durch Alkyl-, Cycloalkyl- oder Arylreste monosubstituiert oder durch Alkyl- oder durch Alkyl- und Cycloalkyl- oder durch Alkyl- und Arylreste disubstituiert ist, durch Cyclisierung von N-Aryl-thioharnstoffen, von N-Aryl-N-alkyl-, N-Aryl-N-cycloalkyl- oder N,N-Diaryl-thioharnstoffen bzw. von N-Aryl-N'-alkyl-, N-Aryl-N'-cycloalkyl-, N,N'-Diaryl-, N-Aryl-N',N'-dialkyl-, N-Aryl-N'-alkyl-N'-cycloalkyl- oder N,N'-Diaryl-N'-alkyl-thioharnstoffen, wobei Alkyl, Cycloalkyl und Aryl substituiertes Alkyl, substituiertes Cycloalkyl bzw. substituiertes Aryl einschließt, das dadurch gekennzeichnet ist, daß man die Cyclisierung mittels Thionylchlorid in einer Menge von mindestens 2 Mol pro Mol Arylthioharnstoff als Ausgangsverbindung durchführt und in dem Falle, in dem die erwähnten N'-substituierten N-Aryl-thioharnstoffe zu den in der 2-ständigen Aminogruppe substituierten 2-Amino-arylenothiazolen umgesetzt werden, man die Arylthioharnstoff-Verbindung zu dem Thionylchlorid zugibt.

Man geht deshalb entsprechend von Verbindungen der Formeln (1A) und (1B)

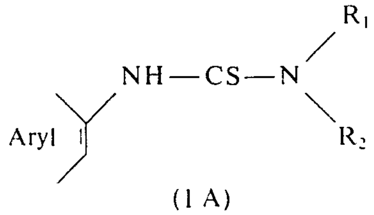

(1 A)

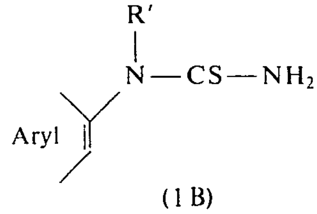

(1 B)

aus und kommt entsprechend zu den Thiazolen bzw. Thiazolinen der Formeln (2 A) und (2 B)

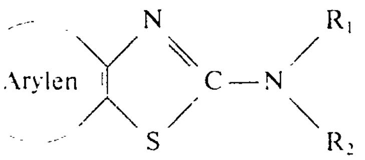

(2 A)

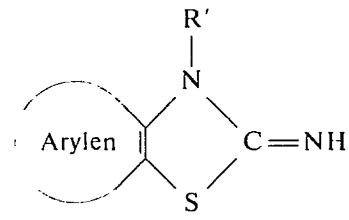

(2 B)

Hierin bedeuten »Aryl« einen monovalenten carbocyclischen oder heterocyclischen aromatischen Rest, der unsubstituiert oder durch Halogenatome, wie Fluor-, Chlor- oder Bromatome, niedere Alkanoyl- und niedere Alkoxycarbonylgruppen als schwach elektronegative Substituenten, durch elektroneutrale und/oder elektropositive Substituenten substituiert ist, »Arylen« einen carbocyclischen oder heterocyclischen aromatischen, ortho-ständig bivalenten Rest, der unsubstituiert oder durch elektroneutrale, elektropositive und/oder die erwähnten schwach elektronegativen Substituenten substituiert ist, $R_1$ ein Wasserstoffatom, einen gegebenenfalls substituierten Alkylrest, einen gegebenenfalls substituierten Cycloalkylrest oder einen gegebenenfalls substituierten Arylrest und $R_2$ ein Wasserstoffatom oder einen gegebenenfalls substituierten Alkylrest, wobei $R_1$ und $R_2$ gleich oder verschieden sind, und R' ein Wasserstoffatom, einen gegebenenfalls substituierten Alkylrest, einen gegebenenfalls substituierten Cycloalkylrest oder einen gegebenenfalls substituierten Arylrest. Die jeweiligen Gruppen $R_1$, $R_2$ und R' in den Endprodukten sind mit denen der Ausgangsverbindungen identisch.

Alkylreste sind bevorzugt niedere Alkylreste. Die Angabe »niedere« bedeutet hier wie im folgenden, daß die Alkyl- oder Alkylenreste in den so bezeichneten Gruppen jeweils 1 bis 6 C-Atome, vorzugsweise 1 bis 4 C-Atome, enthalten.

Die Arylreste bzw. Arylenreste sind bevorzugt Phenyl- und Naphthyl- bzw. Phenylen- und Naphthylenreste. Cycloalkylreste sind bevorzugt der Cyclopentyl- und der Cyclohexylrest, die noch durch 1 bis 3 Methylgruppen substituiert sein können.

Elektropositive oder elektroneutrale Substituenten sind beispielsweise niedere Alkylgruppen, wie Methyl-, Äthyl- oder Propylgruppen, die Hydroxygruppe, niedere Alkoxygruppen, wie Methoxy-, Äthoxy-, Propoxy- oder Butoxygruppen, gegebenenfalls durch niedere, gegebenenfalls substituierte aliphatische Reste oder gegebenenfalls substituierte Arylreste, wie gegebenenfalls substituierte Phenylreste, substituierte Amino- oder Acylaminogruppen, wie beispielsweise Acylaminogruppen von niederen aliphatischen Carbonsäuren oder von Arylcarbonsäuren, wie der Benzoesäure, niedere Alkylmercapto- und Acyloxygruppen von niederen aliphatischen Carbonsäuren oder Arylcarbonsäuren, wie der Benzoesäure.

Dieses erfindungsgemäße Verfahren läuft chemisch in der Weise, daß 2 Mol der Arylthioharnstoff-Verbindung sich mit 4 Mol Thionylchlorid und 2 Mol Wasser zu 2 Mol des 2-Amino-arylenothiazol- bzw. N-substituierten 2-Imino-arylenothiazolin-hydrochlorids bzw. 2-N-substituierten 2-Amino-thiazolhydrochlorids, 3 Mol Schwefeldioxid, 1 Mol Schwefel und 6 Mol Chlorwasserstoff umsetzen. Der Schwefelanfall ist demnach sehr gering, die übrigen Verfahrensprodukte sind leicht abtrennbar.

Das erfindungsgemäße Verfahren verläuft einheitlich und ergibt hohe Ausbeuten an analysenreinen 2-Amino-arylenothiazolen und deren 2-N-substituierten Verbindungen bzw. im Thiazolinring N-substituierten 2-Imino-arylenothiazolin-Verbindungen.

Mit Blick auf das obenerwähnte Verfahren aus Journ. Indian Chem. Soc. 44 war es überraschend, daß sich die »asymmetrischen« Arylthioharnstoffe leicht und in hoher Ausbeute mittels Thionylchlorid in ihre entsprechenden, in der 2-ständigen Aminogruppe unsubstituierten 2-Amino-arylenothiazole bzw. 2-Imino-arylenothiazole überführen lassen und daß in ebenfalls hoher Ausbeute und Reinheit auch die 2-N-substituierten 2-Aminoarylenothiazole mittels Thionylchlorid hergestellt werden können, sofern man die Ausgangs-Thioharnstoffverbindung in das vorgelegte, überschüssige Thionylchlorid zugibt.

Die vorliegende Erfindung betrifft bevorzugt die Herstellung von Verbindungen der Formeln (2A) und (2B), in welchen »Arylen« den Phenylen- oder Naphthylenrest bedeutet, der durch 1, 2 oder 3

elektroneutrale, elektropositive und/oder der erwähnten schwach elektronegativen Substituenten substituiert sein kann, insbesondere durch 1, 2 oder 3 Substituenten aus der Gruppe niederes Alkyl, Hydroxy, niederes Alkoxy, gegebenenfalls durch niederes Alkyl und/oder Phenyl substituiertes Amino, niederes Alkanoylamino, Benzoylamino, niederes Alkylmercapto, niederes Alkanoyl, Benzoyl, Fluor, Chlor, Brom, niederes Alkanoyloxy und niederes Alkoxycarbonyl, $R_1$ und $R_2$ gleich oder verschieden sind und $R_1$ für ein Wasserstoffatom, eine Alkylgruppe von 1 bis 8 C-Atomen, die durch Halogen, wie Fluor, Chlor oder Brom, niederes Alkoxy, wie Methoxy oder Äthoxy, oder durch einen Phenylrest substituiert sein kann, wobei der Phenylrest 1, 2 oder 3 elektroneutrale, elektropositive und/oder der erwähnten schwach elektronegativen Substituenten enthalten kann, oder für einen gegebenenfalls substituierten Cycloalkylrest steht, $R_2$ ein Wasserstoffatom oder eine Alkylgruppe von 1 bis 8 C-Atomen, die durch Halogen, wie Fluor, Chlor oder Brom, durch niederes Alkoxy, wie Methoxy oder Äthoxy, oder durch einen Phenylrest substituiert sein kann, wobei der Phenylrest 1, 2 oder 3 elektroneutrale, elektropositive und/oder der erwähnten schwach elektronegativen Substituenten enthalten kann, oder einen gegebenenfalls substituierten Cycloalkylrest oder gegebenenfalls substituierten Arylrest bedeutet und R' für ein Wasserstoffatom, einen gegebenenfalls substituierten Aryl-, gegebenenfalls substituierten Cycloalkyl- oder gegebenenfalls substituierten Alkylrest steht, indem man von den entsprechenden Verbindungen der Formeln (1A) und (1B) ausgeht, in welchen »Aryl« den Phenyl- oder Naphthylrest bedeutet, der durch 1, 2 oder 3 elektroneutrale, elektropositive und/oder der erwähnten schwach elektronegativen Substituenten substituiert sein kann, insbesondere durch 1, 2 oder 3 Substituenten aus der Gruppe niederes Alkyl, Hydroxy, niederes Alkoxy, gegebenenfalls durch niederes Alkyl und/oder Phenyl substituiertes Amino, niederes Alkanoylamino, Benzoylamino, niederes Alkylmercapto, niederes Alkanoyl, Benzoyl, Fluor, Chlor, Brom, niederes Alkanoyloxy und niederes Alkoxycarbonyl, und $R_1$, $R_2$ und R' die obengenannten Bedeutungen haben, und diese erfindungsgemäß mittels Thionylchlorid cyclisiert, wobei man in dem Falle, in dem eine Verbindung (1A) zur Verbindung (2A), in welchen $R_1$ oder $R_2$ oder beide nicht Wasserstoff sind, umgesetzt wird, die Verbindung (1A) zum Thionylchlorid zugibt.

Bevorzugt ist nach dem erfindungsgemäßen Verfahren insbesondere die Herstellung von 2-Aminobenzthiazol-Verbindungen der allgemeinen Formel (2a) bzw. von 2-Imino-benzothiazolin-Verbindungen der allgemeinen Formel (2b)

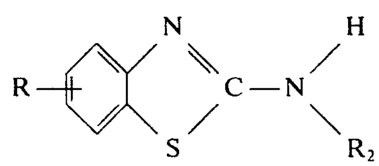

(2a)

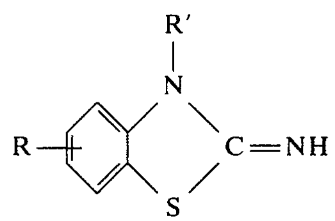

(2b)

in welchen R für ein Wasserstoffatom oder Fluor-, Chlor- oder Bromatom oder eine niedere Alkylgruppe, wie Methyl- oder Äthylgruppe, oder eine niedere Alkoxygruppe, wie Methoxy- oder Äthoxygruppe, eine Hydroxygruppe oder eine Aminogruppe steht, und $R_2$ ein Wasserstoff, eine niedere Alkylgruppe, wie eine Methyl-, Äthyl-, Propyl- oder Butylgruppe, oder eine gegebenenfalls substituierte Cycloalkylgruppe, wie die Cyclohexylgruppe, oder eine gegebenenfalls substituierte Arylgruppe, insbesondere eine Phenylgruppe, die durch eine, zwei oder drei der obengenannten elektroneutralen, elektropositiven und/oder schwach elektronegativen Substituenten substituiert sein kann, beispielsweise durch 1 oder 2 Substituenten aus der Gruppe Methyl, Äthyl, Methoxy, Äthoxy und Chlor, wobei $R_2$ bevorzugt beide Wasserstoff ist, und R' ein Wasserstoffatom oder eine Alkylgruppe von 1 bis 4 C-Atomen bedeutet, die gegebenenfalls durch einen Phenylrest, eine Hydroxygruppe oder eine sekundäre Aminogruppe der Formel $-NR_3R_4$ substituiert sein kann, in welcher $R_3$ und $R_4$ gleich oder verschieden voneinander sein können und $R_3$ eine Alkylgruppe von 1 bis 4 C-Atomen, eine Benzylgruppe oder den Phenylrest bedeutet und $R_4$ eine Alkylgruppe von 1 bis 4 C-Atomen darstellt, oder R' einen gegebenenfalls substituierten Cycloalkylrest, vorzugsweise einen Cyclohexylrest, oder einen gegebenenfalls durch die obengenannten elektroneutralen, elektropositiven und/oder schwach elektronegativen Substituenten substituierten Arylrest, wie Phenylrest, bedeutet; zur Herstellung der Verbindungen der Formeln (2) geht man entsprechend von Thioharnstoff-Verbindungen der allgemeinen Formel (1)

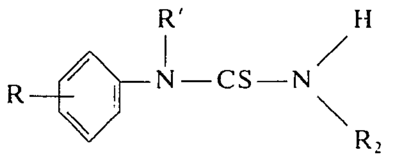

(1)

aus, in welchen R, R' und $R_2$ die obengenannte Bedeutung besitzen, wobei R' zwingend Wasserstoff ist, wenn $R_2$ eine andere Gruppe als Wasserstoff darstellt.

Das erfindungsgemäße Verfahren kann in organischen Lösungsmitteln, wie Toluol, Chlorbenzol oder Dichlorbenzol, durchgeführt werden. Zweckmäßig arbeitet man jedoch in Abwesenheit von organischen Lösungsmitteln unter Verwendung von mindestens der 2fachen molaren Menge an Thionylchlorid, bezogen auf den Arylthioharnstoff als Ausgangsverbindung. Vorteilhaft führt man die Reaktion in Thionylchlorid als Lösungsmittel oder Reaktionsmedium durch, wobei man vorzugsweise pro Mol Arylthioharnstoff der Formel (1) etwa 2,1 bis 5 Mol, insbesondere 2,5 bis 3 Mol Thionylchlorid einsetzt. Es können zwar höhere Überschüsse an Thionylchlorid verwendet werden, jedoch ist dies aus wirtschaftlicher und ökologischer Sicht nicht sinnvoll.

Das erfindungsgemäße Verfahren wird besonders günstig in der Weise durchgeführt, daß man den Ausgangs-Arylthioharnstoff in das Thionylchlorid bei einer Temperatur zwischen 10 und 70°C, vorzugsweise bei einer Temperatur zwischen 10 und 55°C, unter Rühren einträgt und sodann während einer Reaktionszeit von einigen Stunden, wie etwa 3 bis 5 Stunden, die Reaktion bei einer Temperatur zwischen etwa 40 und 80°C, vorzugsweise zwischen 50 und 65°C, durchführt. Man erhält eine Schmelze, die anschließend mit Wasser langsam zersetzt wird, wobei man entweder diese Schmelze mit Wasser versetzt oder diese Schmelze langsam in vorgelegtes Wasser gibt. Man erhält eine wäßrige Lösung des Hydrochlorids der 2-Aminoarylenothiazol- bzw. 2-Imino-arylenothiazolin-Verbindung; der bei der Reaktion gebildete Schwefel suspendiert in dieser Lösung.

Die bei der Cyclisierungsreaktion gemäß dem erfindungsgemäßen Verfahren und der anschließenden Hydrolyse gebildeten Gase aus Schwefeldioxid und Chlorwasserstoff können vorteilhaft zuerst in Wasser, danach in wäßriger Alkalilauge, wie Natronlauge, absorbiert werden. Die hierbei anfallende Salzsäure und die Natriumbisulfitlösung können in zahlreichen technischen Prozessen eingesetzt werden. Diese Abfallprodukte sind somit im technischen Produktionsverbund recyclisierbar und belasten die Ökologiebilanz dieses neuen, erfindungsgemäßen Verfahrens nicht.

Die erhaltene wäßrige Lösung des 2-Aminoarylenothiazol-hydrochlorids bzw. des 2-Imino-arylenothiazolin-hydrochlorids wird nach Abfiltrieren von dem suspendierten Schwefel mittels Aktivkohle geklärt; das 2-Aminoarylenothiazol bzw. 2-Imino-arylenothiazolin wird danach durch Zugabe von einem basischen Neutralisationsmittel, wie Ammoniak oder einem Hydroxid, Oxid oder Carbonat eines Alkali- oder Erdalkalimetalls, wie des Natriums, Kaliums oder Calciums, abgeschieden. Bevorzugt verwendet man hierzu wäßriges Ammoniak. Das abgeschiedene Thiazol bzw. Thiazolin der Formeln (2) wird anschließend durch Abfiltrieren oder Abzentrifugieren isoliert.

Das erfindungsgemäße Verfahren hat weiterhin den Vorteil, daß es in einfachen Apparaturen mit geringem Raumbedarf sowie ohne organisches Lösungsmittel durchgeführt werden kann. Es liefert die Thiazole bzw. Thiazoline der Formeln (2) in hoher Reinheit und Ausbeute. Der bei der Reaktion anfallende Schwefel wird gegenüber technisch gebräuchlichen Verfahren auf weniger als ein Fünftel gesenkt.

Die Ausgangsverbindungen der Formel (1A) können beispielsweise durch Umsetzung entsprechender Arylisothiocyanate (Senföle) mit primären bzw. sekundären Aminen gemäß der Gleichung

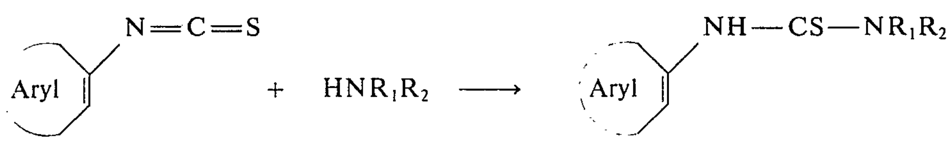

hergestellt werden (vgl. Houben-Weyl, Methoden der organischen Chemie, Band IX, S. 889 ff. [1955]).

Die nachstehenden Beispiele dienen zur Erläuterung der Erfindung. Die Teile sind Gewichtsteile, die Prozentangaben sind Gewichtsprozent, sofern nichts anderes vermerkt.

### Beispiel 1

In 666,4 Teile Thionylchlorid werden bei einer Temperatur zwischen 50 und 60°C innerhalb von 2 Stunden 364 Teile 4-Methoxyphenylthioharnstoff gleichmäßig eingetragen; die erhaltene Suspension wird anschließend 4 Stunden bei der Temperatur zwischen 50 und 60°C gerührt. Die entweichenden Reaktionsgase (Schwefeldioxid und Chlorwasserstoff) werden in eine wäßrige Salzsäurelösung bzw. Natriumsulfitlösung übergeführt.

Man gießt die Cyclisierungsschmelze sodann unter Rühren in eine Mischung aus 2700 Teilen Wasser und 40 Teilen Kieselgur, rührt 30 Minuten bei 60° C, klärt mit 10 Teilen Aktivkohle, filtriert hiervon ab und gibt die Lösung in 350 Teile 25%ige wäßrige Ammoniaklösung. Das 6-Methoxy-2-aminobenzothiazol fällt in farblosen Kristallen aus; man filtriert es ab, wäscht es mit Wasser und trocknet es. Man erhält 354,6 Teile (96,0% d. Th.) 6-Methoxy-2-aminobenzothiazol mit einem Schmelzpunkt von 162°C und einem Reinheitsgehalt von größer als 99,5% gemäß einer potentiometrischen Titration mit Perchlorsäure.

## Beispiel 2

Verfährt man wie in Beispiel 1 beschrieben, setzt jedoch anstelle von 4-Methoxy-phenylthioharnstoff äquimolare Mengen an 4-Äthoxy-phenylthioharnstoff ein, so erhält man das 6-Äthoxy-2-aminobenzothiazol mit einem Schmelzpunkt von 174°C in 95,6%iger Ausbeute d. Th. und in gleich guter Reinheit.

## Beispiel 3

Man trägt innerhalb von 3 Stunden 166 Teile 2-Methyl-phenylthioharnstoff bei 20–30°C gleichmäßig in 298,7 Teile Thionylchlorid ein; das Reaktionsgemisch heizt man in einer Zeit von 2 Stunden auf eine Temperatur von 45 bis 50° C und rührt anschließend 3 Stunden bei dieser Temperatur weiter. Danach tropft man zu dem Reaktionsprodukt innerhalb von 2 Stunden 250 Teile Wasser zu, wobei die Temperatur zwischen 50 und 60° C gehalten wird. Die entweichenden Reaktionsgase werden entsprechend Beispiel 1 absorbiert. Die sich bildende Suspension wird anschließend in eine Mischung aus 1000 Teilen Wasser und 20 Teilen Kieselgur eingerührt, 5 Teile Aktivkohle werden zugesetzt, und das Ganze wird bei etwa 60° C 30 Minuten lang gerührt. Man filtriert anschließend ab und stellt das Filtrat durch Zutropfen von etwa 30%iger Natronlauge auf einen pH von 8,5. Der ausgefallene Niederschlag wird bei 20° C abgesaugt, mit Wasser gewaschen und getrocknet.

Man erhält 155,5 Teile (94,8% d. Th.) 4-Methyl-2-aminobenzothiazol mit einem Schmelzpunkt von 136° C und einem Reingehalt von größer als 99,5%.

Man erhält das 4-Methyl-2-aminobenzothiazol aus der obigen Reaktionsschmelze in gleich guter Ausbeute und Qualität, wenn man die Schmelze in 1250 Teile Wasser, dem 20 Teile Kieselgur zugesetzt werden, eindrückt und ansonst in der angegebenen Weise weiterverfährt.

## Beispiel 4

167 Teile 4-Amino-phenylthioharnstoff werden innerhalb von 4 Stunden unter Rühren in 394,4 Teile etwa 50° C warmes Thionylchlorid eingetragen; man rührt bei etwa 50° C 4 Stunden lang nach und hydrolysiert anschließend die Cyclisierungsschmelze durch tropfenweise Zugabe von 300 Teilen Wasser, wie in Beispiel 3 beschrieben. Die erhaltene Suspension wird 3 Stunden bei etwa 60°C nachgerührt, anschließend in ein Gemisch aus 800 Teilen Wasser und 15 Teilen Kieselgur eingegossen, mit 15 Teilen Aktivkohle versetzt und anschließend filtriert. Das Filtrat wird in 300 Teile 25%ige wäßrige Ammoniaklösung fließen lassen, der ausgefallene farblose Niederschlag abgesaugt, mit wenig Wasser gewaschen und getrocknet.

Man erhält 141,6 Teile (85,7% d. Th.) 2,6-Diamino-benzothiazol mit einem Schmelzpunkt von 207°C und einem Reinheitsgehalt von größer als 95%.

## Beispiel 5

Verfährt man wie in Beispiel 4 beschrieben, verwendet jedoch anstelle von 4-Amino-phenylthioharnstoff äquimolare Mengen an 5-Amino-1-naphthylthioharnstoff, so erhält man entsprechend das 2,6-Diaminonaphtho[1,2-d]thiazol in gleich guter Ausbeute und Qualität.

## Beispiel 6

186,5 Teile 3-Chlor-phenylthioharnstoff werden unter Rühren innerhalb von 90 Minuten in 334,6 Teile etwa 65° C warmes Thionylchlorid eingetragen; die Reaktionsmischung wird danach 5 Stunden unter Rückfluß (65–70° C) gerührt. Die erhaltene Cyclisierungsschmelze wird analog Beispiel 1 mit 3200 Teilen Wasser hydrolysiert und wie dort beschrieben aufgearbeitet. Man erhält 170,3 Teile (entsprechend 92,3% d. Th.) eines Gemisches aus 5- bis 7-Chlor-2-aminobenzothiazol, das keine erkennbaren Verunreinigungen aufweist.

Beispiel 7

Verfährt man wie in Beispiel 6 beschrieben, verwendet jedoch anstelle von 3-Chlor-phenylthioharnstoff gleiche Mengen 4-Chlor-phenylthioharnstoff, so erhält man 173,4 Teile (entsprechend 94,0% d. Th.) 6-Chlor-2-aminobenzothiazol mit einem Schmelzpunkt von 205°C und einem Reinheitsgehalt von 98,9% gemäß potentiometischer Titration mit Perchlorsäure.

Beispiel 8 – 22

Nach dem erfindungsgemäßen Verfahren, wie beispielsweise nach einer in den obigen Beispielen beschriebenen Verfahrensweise, werden auch die in der nachfolgenden Tabelle genannten Verbindungen entsprechend der nachstehenden allgemeinen Formel (3) mit den in der Tabelle angegebenen Ausbeuten und Reinheitsgraden erhalten, wenn man von den entsprechenden Phenylthioharnstoffen der Formel (4) ausgeht:

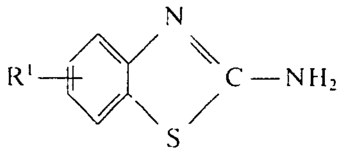

(3)

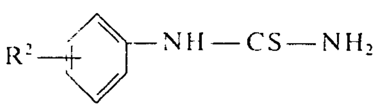

(4)

| Bsp. | $R^1$ | $R^2$ | Ausbeute (% d. Th.) | Rein-heits-grad | Smp. (in °C) |
|---|---|---|---|---|---|
| 8 | H | H | 93,2 | 99,1% | 130 |
| 9 | 6-(i-$C_4H_9$) | 4-(i-$C_4H_9$) | 88,7 | 98,5% | 140 |
| 10 | 4-n-$OC_4H_9$ | 2-n-$OC_4H_9$ | 94,0 | 99,4% | 144 |
| 11 | 6-i-$C_5H_{11}$ | 4-i-$C_5H_{11}$ | 93,2 | 99,5% | 122 |
| 12 | 5- und 7-$CH_3$ | 3-$CH_3$ | 87,0 | – | – |
| 13 | 6-i-$OC_3H_7$ | 4-i-$OC_3H_7$ | 85,5 | 94,9% | 135 |
| 14 | 6-$CH_3$ | 4-$CH_3$ | 96,4 | 99,2% | 140 |
| 15 | 6-NH – CO – $CH_3$ | 4-NH – CO – $CH_3$ | 90,0 | 97,5% | 239 |
| 16 | 6-S – $C_2H_5$ | 4-S – $C_2H_5$ | 92,7 | 99,0% | 171 |
| 17 | 6-CO – O – $C_2H_5$ | 4-CO – O – $C_2H_5$ | 81,2 | 95,2% | 241 |
| 18 | 6-OC – $CH_3$ | 4-OC – $CH_3$ | 93,8 | 98,0% | 178 |
| 19 | 6-CO – O – $CH_3$ | 4-CO – O – $CH_3$ | 79,4 | 98,9% | 200 |
| 20 | 4,5-Benzo | 2,3-Benzo | 92,0 | 98,2% | 186 |
| 21 | 6-Br | 4-Br | 98,1 | 99,0% | 212 |
| 22 | 6-($N(CH_3)_2$ | 4-$N(CH_3)_2$ | 81,7 | 90,1% | 176 |

## Beispiel 23

In 666,4 Teile Thionylchlorid werden bei einer Temperatur zwischen 50 und 60°C innerhalb von 2 Stunden 360 Teile N-Äthyl-N-phenylthioharnstoff gleichmäßig eingetragen; die erhaltene Suspension wird anschließend 4 Stunden bei der Temperatur zwischen 50 und 60°C gerührt. Die entweichenden Reaktionsgase (Schwefeldioxid und Chlorwasserstoff) werden in einer zweistufigen Absorptionsanlage (Wasser und Natronlauge) in eine wäßrige Salzsäurelösung bzw. Natriumbisulfitlösung übergeführt.

Man gießt die Cyclisierungsschmelze sodann unter Rühren in eine Mischung aus 2700 Teilen Wasser und 40 Teilen Kieselgur, rührt 30 Minuten bei 60°C, klärt mit 10 Teilen Aktivkohle, filtriert hiervon ab und gibt die Lösung in 350 Teile 25%ige wäßrige Ammoniaklösung. Das 3-Äthyl-2-iminobenzothiazolin fällt in farblosen Kristallen aus; man filtriert es ab, wäscht es mit Wasser und trocknet es. Man erhält 341,7 Teile (96,0% d. Th.) 3-Äthyl-2-iminobenzothiazolin mit einem Schmelzpunkt von 85°C und einem Reinheitsgehalt von größer als 99,5% gemäß einer potentiometrischen Titration mit Perchlorsäure.

## Beispiel 24

Verfährt man wie in Beispiel 23 beschrieben, setzt jedoch anstelle von N-Äthyl-N-phenylthioharnstoff äquimolare Mengen an N-Methyl-N-phenylthioharnstoff ein, so erhält man das 3-Methyl-2-iminobenzothiazolin mit einem Schmelzpunkt von 122°C in 95,6%iger Ausbeute d. Th. und in gleich guter Reinheit.

## Beispiel 25

Man trägt innerhalb von 3 Stunden 180 Teile N-(2-Methylphenyl)-N-methyl-thioharnstoff bei 20–30°C gleichmäßig in 298,7 Teile Thionylchlorid ein; das Reaktionsgemisch heizt man in einer Zeit von 2 Stunden auf eine Temperatur von 45 bis 50°C und rührt anschließend 3 Stunden bei dieser Temperatur weiter. Danach tropft man zu dem Reaktionsprodukt innerhalb von 2 Stunden 250 Teile Wasser zu, wobei die Temperatur zwischen 50 und 60°C gehalten wird. Die entweichenden Reaktionsgase werden entsprechend Beispiel 23 absorbiert. Die sich bildende Suspension wird anschließend in eine Mischung aus 1000 Teilen Wasser und 20 Teilen Kieselgur eingerührt, 5 Teile Aktivkohle werden zugesetzt, und das Ganze wird bei etwa 60°C 30 Minuten lang gerührt. Man filtriert anschließend ab und stellt das Filtrat durch Zutropfen von etwa 30%iger Natronlauge auf einen pH von 8,5. Der ausgefallene Niederschlag wird bei 20°C abgesaugt, mit Wasser gewaschen und getrocknet.

Man erhält 162,5 Teile (91,3% d. Th.) 3,4-Dimethyl-2-imino-benzothiazolin mit einem Schmelzpunkt von 83,5°C und einem Reingehalt von größer als 98,5%.

Man erhält das 3,4-Dimethyl-2-iminobenzothiazolin aus der obigen Reaktionsschmelze in gleich guter Ausbeute und Qualität, wenn man die Schmelze in 1250 Teile Wasser, dem 20 Teile Kieselgur zugesetzt waren, eindrückt und ansonst in der angegebenen Weise weiterverfährt.

## Beispiel 26

210 Teile N-(4-Methoxyphenyl)-N-äthyl-thioharnstoff werden innerhalb von 4 Stunden unter Rühren in 394,4 Teile etwa 50°C warmes Thionylchlorid eingetragen; man rührt bei etwa 50°C 4 Stunden lang nach und hydrolysiert anschließend die Cyclisierungsschmelze durch tropfenweise Zugabe von 300 Teilen Wasser, wie in Beispiel 25 beschrieben. Die erhaltene Suspension wird 3 Stunden bei etwa 60°C nachgerührt, anschließend in ein Gemisch aus 800 Teilen Wasser und 15 Teilen Kieselgur eingegossen, mit 15 Teilen Aktivkohle versetzt und anschließend filtriert. Das Filtrat wird in 300 Teile 25%ige wäßrige Ammoniaklösung fließen lassen, der ausgefallene farblose Niederschlag abgesaugt, mit wenig Wasser gewaschen und getrocknet.

Man erhält 182,0 Teile (87,5% d. Th.) 6-Methoxy-3-äthyl-2-imino-benzothiazolin mit einem Schmelzpunkt von 60°C und einem Reinheitsgehalt von größer als 95%.

## Beispiel 27

Verfährt man wie in Beispiel 26 beschrieben, verwendet jedoch anstelle von N-(4-Methoxyphenyl)-N-äthyl-thioharnstoff äquimolare Mengen an N-(4-Chlorphenyl)-N-methyl-thioharnstoff, so erhält man entsprechend das 6-Chlor-3-methyl-2-imino-benzothiazolin mit einem Festpunkt von 79°C in gleich guter Ausbeute und Qualität.

Beispiel 28

125,5 Teile N-Phenyl-N-($\beta$-diäthylaminoäthyl)-thioharnstoff werden unter Rühren innerhalb von 90 Minuten in 334,6 Teile etwa 65°C warmes Thionylchlorid eingetragen; die Reaktionsmischung wird danach 5 Stunden unter Rückfluß (65 – 70°C) gerührt. Die erhaltene Cyclisierungsschmelze wird analog Beispiel 23 mit 3200 Teilen Wasser hydrolysiert. Die geklärte wäßrige Lösung scheidet beim Einlaufenlassen in Ammoniak das Endprodukt ölig aus; dessen Vakuumdestillation liefert 102,5 Teile (entsprechend 82,3% d. Th.) 3-($\beta$-Diäthylaminoäthyl)-2-imino-benzothiazolin bei einem Siedepunkt von 190°C bei 0,133 mbar. Es zeigt im Gaschromatogramm keine erkennbaren Verunreinigungen.

Beispiel 29

Verfährt man wie in Beispiel 28 beschrieben, verwendet jedoch an Stelle von N-Phenyl-N-($\beta$-diäthylaminoäthyl)-thioharnstoff äquivalente Mengen von N-(2-Äthoxyphenyl)-N-methyl-thioharnstoff, so erhält man 98,7 Teile (entsprechend 94,0% d. Th.) 4-Äthoxy-3-methyl-2-iminobenzothiazolin mit einem Siedepunkt von 180°C bei 0,066 mbar mit einem Reinheitsgehalt von 98,9% gemäß potentiometrischer Titration mit Perchlorsäure.

Beispiele 30 bis 52

Nach dem erfindungsgemäßen Verfahren, wie beispielsweise nach einer in den obigen Beispielen beschriebenen Verfahrensweise, werden auch die in der nachfolgenden Tabelle genannten Verbindungen entsprechend der nachstehenden allgemeinen Formel (3a) mit den in der Tabelle angegebenen Ausbeuten und Reinheitsgraden erhalten, wenn man von den entsprechenden Phenylthioharnstoffen der Formel (4a) ausgeht:

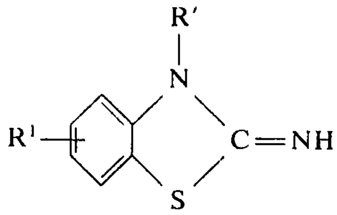

(3a)

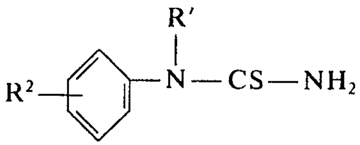

(4a)

| Bsp. | R′ | R¹ | R² | Ausbeute (% d. Th.) | Reinheitsgrad | Smp. oder Sdp. (mbar) |
|---|---|---|---|---|---|---|
| 30 | n-$C_3H_7$ | H | H | 93,1 | 98,9 % | 166°C |
| 31 | n-$C_4H_9$ | H | H | 89,7 | 98,2 % | 157°C (5,33) |
| 32 | i-$C_5H_{11}$ | H | H | 81,0 | 97,9 % | 165°C (4,0) |
| 33 | $-CH_2-CH_2OH$ | H | H | 95,6 | 99,0 % | 120°C |
| 34 | $-CH_2-$ 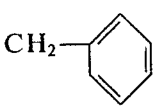 | H | H | 94,1 | 98,0 % | 79°C |
| 35 | Phenyl | H | H | 89,5 | 95,3 % | 208°C |
| 36 | Cyclohexyl | H | H | 82,9 | 98,1 % | 104°C |
| 37 | $-CH_3$ | 6-Br | 4-Br | 93,0 | 95,6 % | 106°C |
| 38 | $-CH_3$ | 6-$CH_3$ | 4-$CH_3$ | 92,5 | 98,3 % | 50°C |
| 39 | $-CH_3$ | 4-$CH_3$ + 6-$CH_3$ | 2-$CH_3$ + 4-$CH_3$ | 88,8 | 97,0 % | 104°C |
| 40 | $-CH_3$ | 6-Phenyl | 4-Phenyl | 97,4 | 92,8 % | 166°C |
| 41 | $-CH_3$ | 6-$OC_2H_5$ | 4-$OC_2H_5$ | 81,0 | 99,4 % | 200°C (0,006) |
| 42 | $-C_2H_5$ | 6-$CH_3$ | 4-$CH_3$ | 91,0 | 96,6 % | 104°C |
| 43 | $-C_2H_5$ | 4-$OCH_3$ | 2-$OCH_3$ | 87,5 | 94,8 % | 40°C |

Fortsetzung.

| Bsp. | R′ | $R^1$ | $R^2$ | Ausbeute (% d. Th.) | Reinheitsgrad | Smp. oder Sdp. (mbar) |
|---|---|---|---|---|---|---|
| 44 | $—C_2H_5$ | $5\text{-}OCH_3$ | $3\text{-}OCH_3$ | 69,0 | 97,8 % | 56°C |
| 45 | $—(CH_2)_2—N(CH_3)_2$ | $6\text{-}OC_2H_5$ | $4\text{-}OC_2H_5$ | 71,4 | 98,9 % | 198°C (0,5) |
| 46 | $—(CH_2)_2—N(CH_3)_2$ | 6-Cl | 4-Cl | 90,0 | 97,8 % | 146°C (0,009) |
| 47 | $—CH_2)_2—N(CH_3)_2$ | $6\text{-}COOC_2H_5$ | $4\text{-}COOC_2H_5$ | 93,9 | 98,7 % | 165°C (0,003) |
| 48 | $—(CH_2)_3—N(CH_3)_2$ | 5-Cl | 3-Cl | 58,9 | 99,2 % | 167°C (1,06) |
| 49 | $—(CH_2)_3—N(CH_3)_2$ | 6-Cl | 4-Cl | 88,2 | 98,2 % | 150°C (0,066) |
| 50 | $—(CH_2)_3—N(CH_3)_2$ | $6\text{-}COOC_2H_5$ | $4\text{-}COOC_2H_5$ | 90,6 | 96,2 % | 168°C (0,0026) |
| 51 | 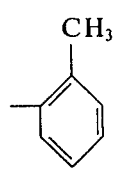 | $4\text{-}CH_3$ | $2\text{-}CH_3$ | 93,9 | 91,9 % | 62°C |
| 52 | 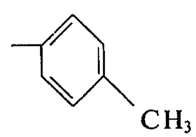 | $6\text{-}CH_3$ | $4\text{-}CH_3$ | 88,7 | 95,0 % | 73°C |

Beispiel 53

In 666,4 Teile Thionylchlorid werden bei einer Temperatur zwischen 50 und 60° C innerhalb von 2 Stunden 328 Teile N'-Methyl-N-phenyl-thioharnstoff gleichmäßig eingetragen; die erhaltene Suspension wird anschließend 4 Stunden bei einer Temperatur zwischen 50 und 60° C gerührt. Die entweichenden Reaktionsgase (Schwefeldioxid und Chlorwasserstoff) werden in einer zweistufigen Absorptionsanlage (Wasser und Natronlauge) in eine wäßrige Salzsäurelösung bzw. Natriumbisulfitlösung übergeführt.

Man gießt die Cyclisierungsschmelze sodann unter Rühren in eine Mischung aus 2700 Teilen Wasser und 40 Teilen Kieselgur, rührt 30 Minuten bei 60° C, klärt mit 10 Teilen Aktivkohle, filtriert hiervon ab und gibt die Lösung in 350 Teile 25%ige wäßrige Ammoniaklösung. Das 2-Methylaminobenzothiazol fällt in farblosen Kristallen aus; man filtriert es ab, wäscht es mit Wasser und trocknet es. Man erhält 310,9 Teile (94,8% d. Th.) 2-Methylamino-benzothiazol mit einem Schmelzpunkt von 137° C und einem Reinheitsgehalt von größer als 99,5% gemäß einer potentiometrischen Titration mit Perchlorsäure.

Beispiel 54

Verfährt man wie in Beispiel 53 beschrieben, setzt jedoch anstelle von N'-Methyl-N-phenyl-thioharnstoff äquimolare Mengen an N'-Äthyl-N-phenyl-thioharnstoff ein, so erhält man das 2-Äthylamino-benzothiazol mit einem Schmelzpunkt von 93° C in 94,4%iger Ausbeute d. Th. und in gleich guter Reinheit.

Beispiel 55

Man trägt innerhalb von 3 Stunden 254 Teile N,N'-Bis-(p-tolyl)-thioharnstoff bei 20–30° C gleichmäßig in 298,7 Teile Thionylchlorid ein; das Reaktionsgemisch heizt man in einer Zeit von 2 Stunden auf eine Temperatur von 45 bis 50° C und rührt anschließend 3 Stunden bei dieser Temperatur weiter. Danach tropft man zu dem Reaktionsprodukt innerhalb von 2 Stunden 250 Teile Wasser zu, wobei die Temperatur zwischen 50 und 60° C gehalten wird. Die entweichenden Reaktionsgase werden entsprechend Beispiel 53 absorbiert. Die sich bildende Suspension wird anschließend in eine Mischung aus 1000 Teilen Wasser und 20 Teilen Kieselgur eingerührt, 5 Teile Aktivkohle werden zugesetzt, und das Ganze wird bei etwa 60° C 30 Minuten lang gerührt. Man filtriert anschließend ab und stellt das Filtrat durch Zutropfen von etwa 30%iger Natronlauge auf einen pH von 8,5. Der ausgefallene Niederschlag wird bei 20° C abgesaugt, mit Wasser gewaschen und getrocknet.

Man erhält 233,4 Teile (91,9% d. Th.) 6-Methyl-2-(p-tolyl-amino)-benzothiazol mit einem Schmelzpunkt von 169° C und einem Reingehalt von größer als 99,5%.

Man erhält das 6-Methyl-2-(p-tolylamino)-benzothiazol aus der obigen Reaktionsschmelze in gleich guter Ausbeute und Qualität, wenn man die Schmelze in 1250 Teile Wasser, dem 20 Teile Kieselgur zugesetzt werden, eindrückt und ansonst in der angegebenen Weise weiterverfährt.

Beispiel 56

178 Teile N',N'-Dimethyl-N-phenyl-thioharnstoff werden innerhalb von 4 Stunden unter Rühren in 394,4 Teile etwa 50° C warmes Thionylchlorid eingetragen; man rührt bei etwa 50° C 4 Stunden lang nach und hydrolysiert anschließend die Cyclisierungsschmelze durch tropfenweise Zugabe von 300 Teilen Wasser, wie in Beispiel 3 beschrieben. Die erhaltene Suspension wird 3 Stunden bei etwa 60° C nachgerührt, anschließend in ein Gemisch aus 800 Teilen Wasser und 15 Teilen Kieselgur eingegossen, mit 15 Teilen Aktivkohle versetzt und anschließend filtriert. Das Filtrat wird in 300 Teile 25%ige wäßrige Ammoniaklösung fließen lassen, der ausgefallene farblose Niederschlag abgesaugt, mit wenig Wasser gewaschen und getrocknet.

Man erhält 169,1 Teile (95,0% d. Th.) 2-(Dimethylamino)-benzothiazol mit einem Schmelzpunkt von 86° C und einem Reinheitsgehalt von größer als 95%.

Beispiel 57

Verfährt man wie in Beispiel 56 beschrieben, verwendet jedoch anstelle von N',N'-Dimethyl-N-phenyl-thioharnstoff äquimolare Mengen an N',N'-Dimethyl-N-(1-naphthyl)-thioharnstoff, so erhält man entsprechend das 2-(Dimethylamino)-naphtho[1,2-d]thiazol mit einem Schmelzpunkt von 136–138° C in gleich guter Ausbeute und Qualität.

Beispiel 58

240,0 Teile N'-Benzyl-N-phenyl-thioharnstoff werden unter Rühren innerhalb von 90 Minuten in 334,6 Teile etwa 65°C warmes Thionylchlorid eingetragen; die Reaktionsmischung wird danach 5 Stunden unter Rückfluß (65–70°C) gerührt. Die erhaltene Cyclisierungsschmelze wird analog Beispiel 53 mit 3200 Teilen Wasser hydrolysiert und wie dort beschrieben aufgearbeitet. Man erhält 221,5 Teile (=92,3% d. Th.) 2-Benzylamino-benzothiazol mit einem Schmelzpunkt von 159°C.

Beispiel 59

Verfährt man wie in Beispiel 58 beschrieben, verwendet jedoch anstelle von N'-Benzyl-N-phenyl-thioharnstoff äquivalente Mengen (232,0 Teile) von N'-Cyclohexyl-N-phenyl-thioharnstoff, so erhält man 218,1 Teile (entsprechend 94,0% d. Th.) 2-Cyclohexylamino-benzothiazol mit einem Schmelzpunkt von 95°C und einem Reinheitsgehalt von 98,9% gemäß potentiometrischer Titration mit Perchlorsäure.

Beispiel 60 bis 88

Nach dem erfindungsgemäßen Verfahren, wie beispielsweise nach einer in den obigen Beispielen beschriebenen Verfahrensweise, werden auch die in der nachfolgenden Tabelle genannten Verbindungen entsprechend der nachstehenden allgemeinen Formel (3b) mit den in der Tabelle angegebenen Ausbeuten und Reinheitsgraden erhalten, wenn man von den entsprechenden Arylthioharnstoffen der Formel (4b) ausgeht:

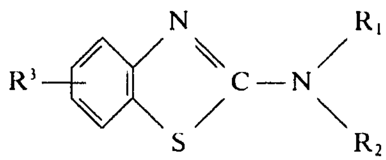

(3 b)

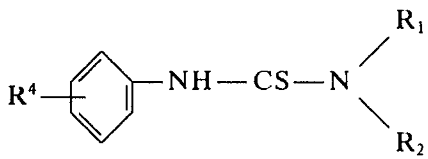

(4 b)

| Beispiel | $R^3$ | $R^4$ | $R_1$ | $R_2$ | Ausbeute (% d. Th.) | Reinheitsgrad | Smp. |
|---|---|---|---|---|---|---|---|
| 60 | H | H | $-C_2H_5$ | H | 93,2 | 99,1 % | 93°C |
| 61 | H | H | n-$C_3H_7$ | H | 88,7 | 98,5 % | 68°C |
| 62 | H | H | n-$C_4H_9$ | H | 94,0 | 99,4 % | 86°C |
| 63 | H | H | i-$C_4H_9$ | H | 93,2 | 99,5 % | 101°C |
| 64 | H | H | n-$C_6H_{13}$ | H | 87,0 | 96,7 % | 64°C |
| 65 | H | H | Phenyl | H | 95,8 | 94,9 % | 161°C |
| 66 | H | H | 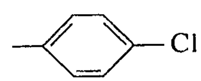 | H | 96,4 | 99,2 % | 197°C |
| 67 | 4-F | 6-F | 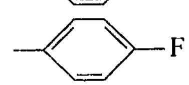 | H | 90,0 | 97,5 % | 225°C |
| 68 | 4-Cl | 6-Cl | 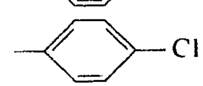 | H | 92,7 | 99,0 % | 223°C |
| 69 | 4-Br | 6-Br | $-CH_3$ | H | 81,2 | 95,2 % | 225°C |
| 70 | 4-Br | 6-Br | $-C_2H_5$ | H | 93,8 | 98,0 % | 157°C |
| 71 | 4-Br | 6-Br | n-$C_4H_9$ | H | 79,4 | 98,9 % | 118°C |
| 72 | 4-Br | 6-Br | 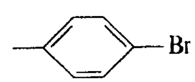 | H | 92,0 | 98,2 % | 257°C |

Fortsetzung

| Beispiel | $R^3$ | $R^4$ | $R_1$ | $R_2$ | Ausbeute (% d. Th.) | Reinheitsgrad | Smp. |
|---|---|---|---|---|---|---|---|
| 73 | 4-Cl | 6-Cl | $-CH_3$ | $-CH_3$ | 98,1 | 99,0% | 99°C |
| 74 | 4-Br | 6-Br | $-CH_3$ | $-CH_3$ | 81,7 | 90,1% | 166°C |
| 75 | 4-$CH_3$ | 6-$CH_3$ | $-CH_3$ | $-CH_3$ | 94,0 | 92,8% | 86°C |
| 76 | 2-$CH_3$ | 4-$CH_3$ | n-$C_3H_7$ | H | 85,8 | 93,0% | 62°C |
| 77 | 2,3-Benzo | 4,5-Benzo | $-C_2H_5$ | H | 92,7 | 90,8% | 106°C |
| 78 | 2,3-Benzo | 4,5-Benzo | n-$C_4H_9$ | H | 98,7 | 92,1% | 67°C |
| 79 | H | H | $-(CH_2)_3-OCH_3$ | H | 79,9 | 98,5% | 88°C |
| 80 | H | H | $-(CH_2)_2-Cl$ | H | 87,0 | 92,3% | 59°C |
| 81 | 4-$OCH_3$ | 6-$OCH_3$ | $-CH_3$ | H | 91,9 | 96,5% | 180°C |
| 82 | 4-$OCH_3$ | 6-$OCH_3$ | $-CH_3$ | $-CH_3$ | 90,2 | 97,1% | 143°C |
| 83 | 4-$OC_2H_5$ | 6-$OC_2H_5$ | $-CH$ | H | 87,8 | 96,5% | 145°C |
| 84 | 4-$OC_2H_5$ | 6-$OC_2H_5$ | $-CH_3$ | $-CH_3$ | 92,1 | 96,3% | 118°C |
| 85 | 4-$OC_2H_5$ | 6-$OC_2H_5$ | n-$C_4H_9$ | H | 88,0 | 95,7% | 100°C |
| 86 | 4-OH | 6-OH | $-CH_3$ | $-CH_3$ | 89,7 | 90,3% | 255°C |
| 87 | 4-OH | 6-OH | $-C_2H_5$ | $-C_2H_5$ | 90,2 | 92,0% | 145°C |
| 88 | 4-$OC_4H_9$(n) | 6-$OC_4H_9$(n) | $-CH_3$ | H | 93,5 | 96,8% | 116°C |

Beispiel 89

182 Teile 4-Methoxyphenylthioharnstoff werden in 900 Teilen Toluol suspendiert und im Verlaufe einer Stunde bei 20–30°C mit 310 Teilen Thionylchlorid versetzt. Man erwärmt nun unter Rühren innerhalb 2 Stunden gleichmäßig ansteigend auf 110°C, ruhrt 2 Stunden bei dieser Temperatur unter Rückfluß nach und destilliert anschließend, nach vorheriger Zugabe von 20 Teilen Kieselgur, das Toluol durch Einblasen von Wasserdampf ab. Nach Zusatz von 5 Teilen Aktivkohle wird die erhaltene Lösung des 6-Methoxy-2-aminobenzothiazol-hydrochlorids geklärt und das Klarfiltrat zu 175 Teilen einer 25%igen wäßrigen Ammoniaklösung gegeben.

Das 6-Methoxy-2-aminobenzothiazol fällt in farblosen Kristallen aus; es wird abfiltriert, mit Wasser gewaschen und getrocknet. Man erhält 173 Teile (96,1% d. Th.) 6-Methoxy-2-aminobenzothiazol vom Schmelzpunkt 162°C und einem Reingehalt von größer als 99,5% (bestimmt durch potentiometrische Titration).

Beispiel 90

Verwendet man in Beispiel 89 an Stelle von Toluol entsprechende Mengen an Chlorbenzol, Äthylenchlorid oder Xylol, so erhält man eine Benzthiazolverbindung in gleich guten Ausbeuten und Qualitäten.

Beispiel 91

Geht man in Beispiel 89 oder 90 an Stelle des 4-Methoxyphenylthioharnstoffes von N-Phenyl-N-methylthioharnstoff in aliquoten Mengen aus und arbeitet sonst in der angegebenen Weise, so erhält man das 3-Methyl-2-imino-benzothiazolin (Fp. 122°C) in ähnlich guter Ausbeute und hohem Reingehalt.

**Patentansprüche**

1. Verfahren zur Herstellung von 2-Amino-arylenothiazolen, die in der 2-ständigen Aminogruppe unsubstituiert sind, oder von im Thiazolinring durch Alkyl, Cycloalkyl oder Aryl N-substituierten 2-Imino-arylenothiazolinen oder von 2-Amino-arylenothiazolen, deren 2-ständige Aminogruppe durch Alkyl, Cycloalkyl oder Aryl monosubstituiert oder durch Alkyl oder durch Alkyl und Cycloalkyl oder durch Alkyl und Aryl disubstituiert ist, durch Cyclisierung von N-Aryl-thioharnstoffen, N-Aryl-N'-alkyl-, N-Aryl-N'-cycloalkyl-, N,N'-Diaryl-, N-Aryl-N',N'-dialkyl-, N-Aryl-N'-alkyl-N'-cycloalkyl-, N,N'-Diaryl-N'-alkyl-, N,N-Diaryl-, N-Aryl-N-alkyl- oder N-Aryl-N-cycloalkyl-thioharnstoffen, wobei die Bezeichnung Alkyl, Cycloalkyl und Aryl substituiertes Alkyl, substituiertes Cycloalkyl bzw. substituiertes Aryl einschließt, dadurch gekennzeichnet, daß man die Cyclisierung mittels Thionylchlorid in einer Menge von mindestens 2 Mol pro Mol Arylthioharnstoff als Ausgangsverbindung durchführt und in dem Falle, in dem die erwähnten N'-substituierten N-Arylthioharnstoffe zu den in der 2-ständigen Aminogruppe substituierten 2-Amino-arylenothiazolen umgesetzt werden, man die Arylthioharnstoff-Verbindung zu dem Thionylchlorid zugibt.

2. Verfahren nach Anspruch 1, dadurch gekennzeichnet, daß man die Cyclisierungsreaktion in Abwesenheit eines organischen Lösungsmittels durchführt.

3. Verfahren nach Anspruch 1 oder 2, dadurch gekennzeichnet, daß man die Cyclisierung bei einer Temperatur zwischen 40 und 80°C durchführt.

4. Verfahren nach einem der Ansprüche 1 oder 3, dadurch gekennzeichnet, daß man das Thionylchlorid in einer Menge von 2,1 bis 5 Mol, bezogen auf 1 Mol Ausgangs-Arylthioharnstoff, verwendet.

5. Verfahren nach einem der Ansprüche 1 bis 4, bei welchem man eine Verbindung der allgemeinen Formel

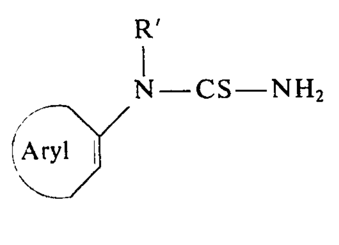

in welcher »Aryl« einen monovalenten carbocyclischen oder heterocyclischen aromatischen Rest, der unsubstituiert oder durch Halogenatome, Alkanoylgruppen von 1 bis 6 C-Atomen im Alkylrest und

Alkoxycarbonylgruppen von 1 bis 6 C-Atomen im Alkylrest als schwach elektronegative Substituenten, durch elektroneutrale und/oder elektropositive Substituenten substituiert ist, bedeutet und R' ein Wasserstoffatom, ein gegebenenfalls substituierter Alkylrest, ein gegebenenfalls substituierter Cycloalkylrest oder ein gegebenenfalls substituierter Arylrest ist, in eine Verbindung der allgemeinen Formel

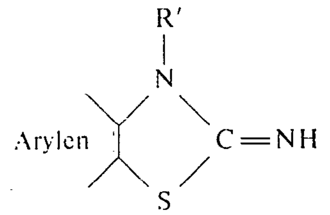

in welcher »Arylen« einen carbocyclischen oder heterocyclischen aromatischen, ortho-ständig bivalenten Rest, der unsubstituiert oder durch elektroneutrale, elektropositive und/oder die obenerwähnten schwach elektronegativen Substituenten substituiert ist, bedeutet und R' die obige Bedeutung besitzt, überführt.

6. Verfahren nach einem der Ansprüche 1 bis 4, bei welchem man eine Verbindung der allgemeinen Formel

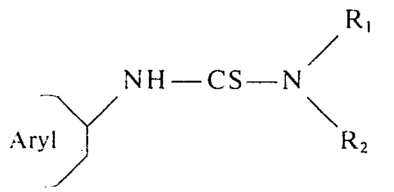

in welcher »Aryl« die im Anspruch 5 gegebene Bedeutung hat, $R_1$ ein Wasserstoffatom, einen gegebenenfalls substituierten Alkylrest, einen gegebenenfalls substituierten Cycloalkylrest oder einen gegebenenfalls substituierten Arylrest und $R_2$ ein Wasserstoffatom oder einen gegebenenfalls substituierten Alkylrest darstellt, wobei $R_1$ und $R_2$ zueinander gleich oder voneinander verschieden sind, in eine Verbindung der allgemeinen Formel

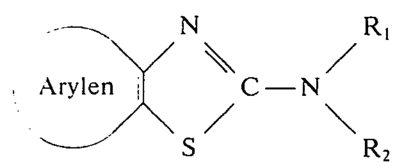

in welcher »Arylen« die im Anspruch 5 gegebene Bedeutung hat und $R_1$ und $R_2$ die obengenannten Bedeutungen besitzen, überführt, wobei die im Anspruch 1 genannte Verfahrensweise der Zugabe der Arylthioharnstoff-Verbindung zu dem Thionylchlorid angewendet wird, falls $R_1$ und $R_2$ beide nicht gleichzeitig Wasserstoffatome bedeuten.

7. Verfahren nach Anspruch 5, bei welchem der Formelrest R' ein Wasserstoffatom oder eine Alkylgruppe von 1 bis 4 C-Atomen bedeutet, die durch einen Phenylrest, durch eine Hydroxygruppe oder eine sekundäre Aminogruppe der Formel $-NR_3R_4$ substituiert sein kann, in welcher $R_3$ und $R_4$ gleich oder verschieden voneinander sein können und $R_3$ eine Alkylgruppe von 1 bis 4 C-Atomen, die Benzylgruppe oder den Phenylrest bedeutet und $R_4$ eine Alkylgruppe von 1 bis 4 C-Atomen darstellt, oder bei welchem R' einen gegebenenfalls substituierten Cycloalkylrest oder einen gegebenenfalls durch Halogenatome, Alkanoylgruppen von 1 bis 6 C-Atomen im Alkylrest und Alkoxycarbonylgruppen von 1 bis 6 C-Atomen im Alkylrest als schwach elektronegative Substituenten, durch elektroneutrale und/oder elektropositive Substituenten substituierten Arylrest bedeutet.

8. Verfahren nach einem der Ansprüche 1 bis 4, bei welchem man eine Verbindung der allgemeinen Formel

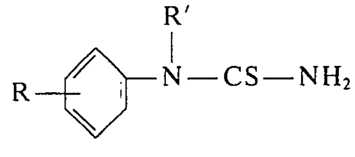

in welcher R' ein Wasserstoffatom oder eine Alkylgruppe bedeutet, die durch einen gegebenenfalls substituierten Arylrest substituiert sein kann und R für ein Wasserstoffatom oder ein Fluor-,

Chlor- oder Bromatom oder eine $C_1-C_6$-Alkylgruppe, $C_1-C_6$-Alkoxygruppe, eine Hydroxygruppe oder eine Aminogruppe steht, in eine Verbindung der allgemeinen Formel

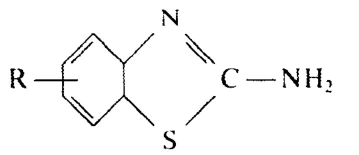

oder

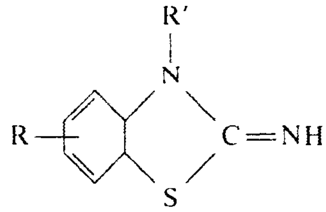

in welchen R' und R die obengenannten Bedeutungen haben, überführt.

9. Verfahren nach einem der Ansprüche 1 bis 4, bei welchem man eine Verbindung der Formel

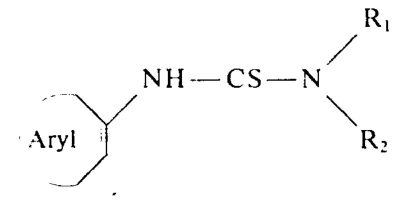

in welcher $R_1$ und $R_2$ gleich oder verschieden sind und $R_1$ ein Wasserstoffatom, eine Alkylgruppe von 1 bis 8 C-Atomen, die durch Halogen, $C_1-C_6$ Alkoxy oder einen Phenylrest substituiert sein kann, wobei der Phenylrest 1, 2 oder 3 Halogenatome, Alkanoylgruppen von 1 bis 6 C-Atomen im Alkylrest und Alkoxycarbonylgruppen von 1 bis 6 C-Atomen im Alkylrest als schwach elektronegative Substituenten, elektroneutrale und/oder elektropositive Substituenten enthalten kann, oder eine Cycloalkylgruppe bedeutet, $R_2$ eine Alkylgruppe von 1 bis 8 C-Atomen darstellt, die durch Halogen, niederes Alkoxy oder einen Phenylrest substituiert sein kann, wobei der Phenylrest 1, 2 oder 3 Halogenatome, niedere Alkanoyl- und niedere Alkoxycarbonylgruppen als schwach elektronegative Substituenten, elektroneutrale und/oder elektropositive Substituenten enthalten kann, oder einen gegebenenfalls substituierten Cycloalkyl- oder einen gegebenenfalls substituierten Arylrest bedeutet und »Aryl« die im Anspruch 5 gegebene Bedeutung besitzt, in eine Verbindung der Formel

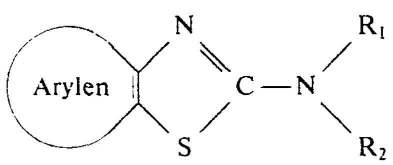

in welcher $R_1$ und $R_2$ die obengenannten Bedeutungen haben und »Arylen« die in Anspruch 5 gegebene Bedeutung hat, überführt.

10. Verfahren nach einem der Ansprüche 1 bis 4, bei welchem man eine Verbindung der Formel

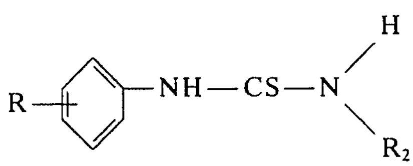

in welcher $R_2$ eine $C_1-C_6$-Alkylgruppe, eine Cycloalkylgruppe oder eine gegebenenfalls substituierte Arylgruppe bedeutet und R ein Wasserstoffatom oder ein Fluor-, Chlor- oder Bromatom oder eine $C_1-C_6$-Alkylgruppe, $C_1-C_6$-Alkoxygruppe, eine Hydroxygruppe oder Aminogruppe ist, in eine Verbindung der Formel

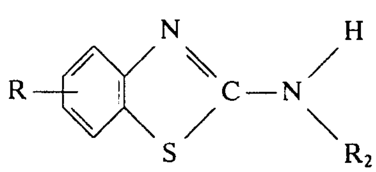

in welcher R und $R_2$ die obengenannte Bedeutung haben, überführt.

**Claims**

1. Process for the manufacture of 2-amino-arylenothiazoles unsubstituted in the amino group in 2-position, or of 2-imino-aryleno-thiazolines N-substituted in the thiazoline ring by alkyl, cycloalkyl or aryl, or of 2-amino-arylenothiazoles which amino group in the 2-position is monosubstituted by alkyl, cycloalkyl or aryl or disubstituted by alkyl or by alkyl and cycloalkyl or by alkyl and aryl, by the way of cyclization of N-aryl-thioureas, N-aryl-N'-alkyl-, N-aryl N'-cycloalkyl-, N,N'-diaryl-, N-aryl-N',N'-dialkyl-, N-aryl-N'-alkyl-N'-cycloalkyl-, N,N'-diaryl-N'-alkyl-, N,N-diaryl-, N-aryl-N-alkyl- or N-aryl-N-cycloalkyl-thioureas, the terms alkyl, cycloalkyl and aryl herein including substituted alkyl, substituted cycloalkyl and, respectively, substituted aryl characterized by that the cyclization is carried out by means of thionyl chloride in an amount of at least 2 mols per mol arylthiourea as starting compound, and in case the mentioned N'-substituted N-arylthioureas are converted to 2-amino-arylenothiazoles substituted in the amino group in 2-position, the arylthiourea-compound is added to the thionyl chloride.

2. Process according to claim 1, characterized by that the cyclization reaktion is carried out in the absence of an organic solvent.

3. Process according to claim 1 or 2, characterized by that the cyclization is carried out at a temperature between 40 and 80° C.

4. Process according to claim 1 or 3, characterized by that the thionyl chloride is used in an amount of from 2.1 to 5 mols for each mol of starting arylthiourea used.

5. Process according to one of claims 1 to 4, wherein a compound of the general formula

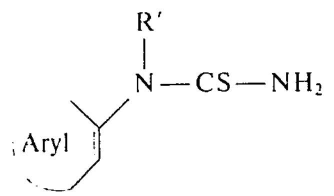

in which »Aryl« means a monovalent carbocyclic or heterocyclic aromatic residue which is unsubstituted or substituted by halogen atoms, alkanoyl groups with 1 to 6 C-atoms in the alkyl radical and alkoxycarbonyl groups with 1 to 6 C-atoms in the alkyl radical as weakly electro-negative substituents, by electro-neutral and/or electro-positive substituents, and R' is a hydrogen atom, an optionally substituted alkyl radical, an optionally substituted cycloalkyl radical or an optionally substituted aryl radical, is converted into a compound of the general formula

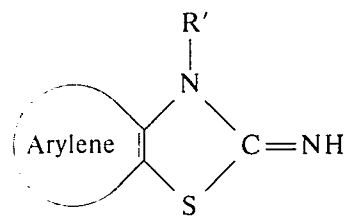

in which »Arylene« means a carbocyclic or heterocyclic aromatic, in ortho-position bivalent, radical which is unsubstituted or substituted by electro-neutral, electro-positive and/or the above mentioned weakly electro-negative substituents, and R' has the above mentioned meaning.

6. Process according to one of claims 1 to 4, wherein a compound of the general formula

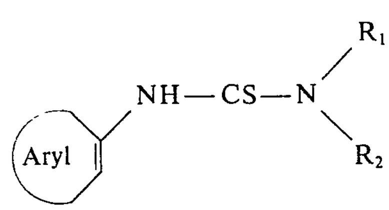

in which »Aryl« has the meaning given in claim 5, $R_1$ is a hydrogen atom, an optionally substituted alkyl radical, an optionally substituted cycloalkyl radical or an optionally substituted aryl radical and $R_2$ is a hydrogen atom or an optionally substitued alkyl radical, the $R_1$ and $R_2$ have each the same meaning or are different from one another, is converted into a compound of the general formula

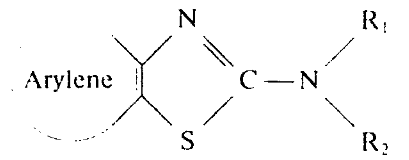

in which »Arylene« has the meaning given in claim 5, and $R_1$ and $R_2$ have the above-mentioned meanings, with the proviso that the process variant of addition of the arylthiourea-compound to the thionyl chloride is used if $R_1$ and $R_2$ both are not simultaneously hydrogen atoms.

7. Process according to claim 5 wherein the formula member R′ means a hydrogen atom or an alkyl group of 1 to 4 C-atoms which may be substituted by a phenyl radical, ty a hydroxy group or a secondary amino group of the formula $-NR_3R_4$ in which $R_3$ and $R_4$ may be the same or different from one another and $R_3$ means an alkyl group of 1 to 4 C-atoms, the benzyl group or the phenyl radical and $R_4$ is an alkyl group of 1 to 4 C-atoms, or wherein R′ means an optionally substituted cycloalkyl radical or an aryl radical optionally substituted by halogen atoms, alkanoyl groups with 1 to 6 C-atoms in the alkyl radical and alkoxycarbonyl groups with 1 to 6 C-atoms in the alkyl radical as weakly electro-negative substituents, by electro-neutral and/or electro-positive substituents.

8. Process according to one of claims 1 to 4 wherein a compound of the general formula

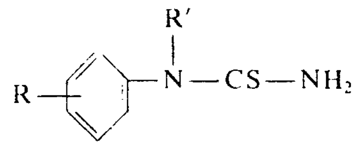

in which R′ means a hydrogen atom or an alkyl group which may be substituted by an optionally substituted aryl radical, and R stands for a hydrogen atom or a fluorine, chlorine or bromine atom or a $C_1-C_6$-alkyl group, $C_1-C_6$-alkoxy group, a hydroxy group or an amino group, is converted into a compound of the general formula

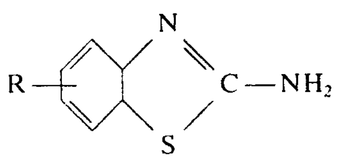 or 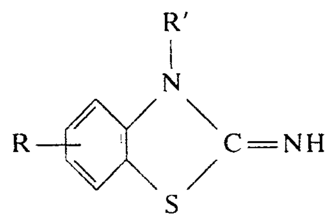

in which R′ and R have the above-mentioned meanings.

9. Process according to one of claims 1 to 4, wherein a compound of the formula

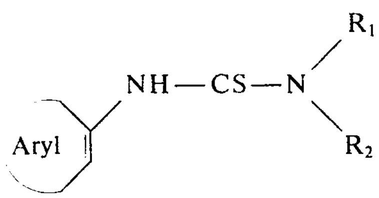

in which $R_1$ and $R_2$ are the same or different from one another and $R_1$ means a hydrogen atom, an alkyl group of 1 to 8 C-atoms which may by substituted by halogen, $C_1-C_6$-alkoxy or a phenyl radical, this phenyl radical may contain 1, 2 or 3 halogen atoms, alkanoyl groups with 1 to 6 C-atoms in the alkyl radical and alkoxycarbonyl groups with 1 to 6 C-atoms in the alkyl radical as weakly electro-negative substituents, electro-neutral and/or electro-positive substituents, or means a cycloalkyl group, $R_2$ is an alkyl group of 1 to 8 C-atoms which may be substituted by halogen, lower alkoxy or a phenyl radical, this phenyl radical may contain 1, 2 or 3 halogen atoms, lower alkanoyl and lower alkoxy carbonyl groups as weakly electro-negative substituents, electro-neutral and/or electro-positive substituents, or means an optionally substituted cycloalkyl or an optionally substituted aryl radical, and »Aryl« has the meaning given in claim 5, is converted into a compound of the formula

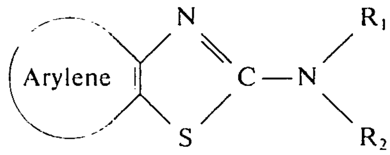

in which $R_1$ and $R_2$ have the above-mentioned meanings and »Arylene« has the meaning given in claim 5.

10. Process according to one of claims 1 to 4 wherein a compound of the formula

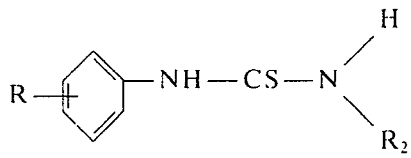

in which $R_2$ means a $C_1-C_6$-alkyl group, a cycloalkyl group or an optionally substituted aryl group and R is a hydrogen atom or a fluorine, chlorine or bromine atom or a $C_1-C_6$-alkyl group, $C_1-C_6$-alkoxy group, a hydroxy group or amino group, is converted into a compound of the formula

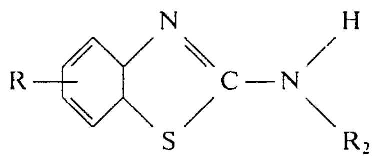

in which R and $R_2$ have the above-mentioned meaning.

**Revendications**

1. Procédé de préparation d'amino-2 arylénothiazoles dont le groupe amino en position 2 est dépourvu de substituant, ou d'imino-2 arylénothiazolines dont l'atome d'azote du cycle thiazolinique porte un alkyle, un cycloalkyle ou un aryle, ou d'amino-2 arylénothiazoles dont le groupe amino en position 2 porte un seul radical alkyle, cycloalkyle ou aryle ou porte deux substituants qui sont deux alkyles, ou un alkyle et un cycloalkyle, ou un alkyle et un aryle, par cyclisation de N-aryl-thio-urées, de N-aryl-N'-alkyl-thio-urées, de N-aryl-N'-cycloalkyl-thio-urées, de N,N'-diaryl-thio-urées, de N-aryl-N',N'-dialkyl-thio-urée, de N-aryl-N'-alkyl-N'-cycloalkyl-thio-urées, de N,N'-diaryl-N'-alkyl-thio-urées, de N,N-diaryl-thio-urées, de N-aryl-N-alkyl-thio-urées ou de N-aryl-N-cycloalkyl-thio-urées, chacun des radicaux alkyles, cycloalkyles et aryles mentionnés pouvant porter un ou plusieurs substituants, procédé caractérisé en ce qu'on effectue la cyclisation au moyen du chlorure de thionyle en une quantité d'au moins 2 moles par mole de l'aryl-thio-urée utilisée comme corps de départ, et dans le cas où l'on transforme les N-aryl-thio-urées substituées en N' mentionnées en les amino-2 arylénothiazoles à groupe amino en 2 substitué, on ajoute l'arylthio-urée au chlorure de thionyle.

2. Procédé selon la revendication 1, caractérisé en ce qu'on effectue la réaction de cyclisation en l'absence de solvant organique.

3. Procédé selon l'une des revendications 1 et 2, caractérisé en ce qu'on effectue la cyclisation à une température comprise entre 40 et 80° C.

4. Procédé selon l'une quelconque des revendications 1 à 3, caractérisé en ce qu'on utilise le chlorure de thionyle en une quantité de 2,1 à 5 moles par mole d'arylthio-urée de départ.

5. Procédé selon l'une quelconque des revendications 1 à 4, selon lequel on transforme un composé répondant à la formule générale

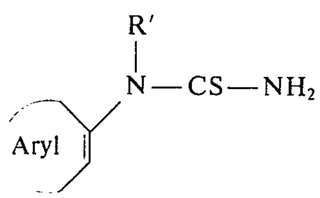

dans laquelle »Aryl« représente un radical aromatique monovalent carbocyclique ou hétérocyclique, qui est dépourvu de substituant ou qui porte des atomes d'halogènes, des radicaux alcanoyles

contenant de 1 à 6 atomes de carbone dans la partie alkylique et des radicaux alcoxycarbonyles contenant de 1 à 6 atomes de carbone dans la partie alkylique, en tant que substituants faiblement électronégatifs, des substituants électriquement neutres et/ou des substituants électropositifs, et R' représente un atome d'hydrogène, un radical alkyle éventuellement substitué, un radical cycloalkyle éventuellement substitué ou un radical aryle éventuellement substitué, en un composé répondant à la formule générale:

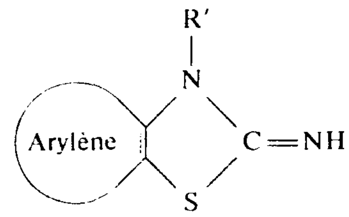

dans laquelle »Arylène« représente un radical aromatique carbocyclique ou hétérocyclique bivalent dont les deux valences sont en ortho l'une par rapport à l'autre, qui est dépourvu de substituant ou qui porte des substituants électriquement neutres et/ou électropositifs et/ou les substituants faiblement électronégatifs qui ont été mentionnés ci-dessus, et R' a la signification précédemment donnée.

6. Procédé selon l'une quelconque des revendications 1 à 4, selon lequel on transforme un composé repondant à la formule générale:

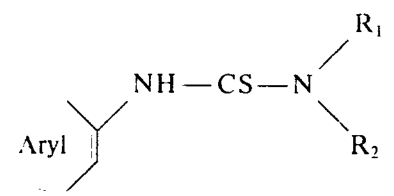

dans laquelle »Aryl« a la signification connée à la revendication 5, $R_1$ représente un atome d'hydrogène, un alkyle éventuellement substitué, un cycloalkyle éventuellement substitué ou un aryle éventuellement substitué, et $R_2$ représente un atome d'hydrogène ou un alkyle éventuellement substitué, $R_1$ et $R_2$ pouvant être identiques l'un à l'autre ou différents l'un de l'autre, en un composé répondant à la formule générale:

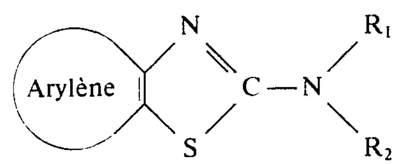

dans laquelle »Arylène« a la signification qui a été donnée à la revendication 5, et $R_1$ et $R_2$ ont les significations précédemment données, le mode opératoire consistent à ajouter l'arylthio-urée au chlorure de thionyle (voir la revendication 1) étant appliqué dans le cas où $R_1$ et $R_2$ ne représentent pas tous les deux en même temps des atomes d'hydrogène.

7. Procédé selon la revendication 5, dans lequel le symbole R' représente un atome d'hydrogène ou un alkyle en $C_1-C_4$ qui peut porter un phényle, un hydroxy ou un amino secondaire $-NR_3R_4$ dans lequel $R_3$ et $R_4$ sont identiques ou différents, $R_3$ représentant un alkyle en $C_1-C_4$, un benzyle ou un phenyle et $R_4$ un alkyle en $C_1-C_4$, ou dans lequel R' représente un cycloalkyle éventuellement substitué ou un aryle éventuellement porteur d'atomes d'halogènes, de radicaux alcanoyles contenant de 1 à 6 atomes de carbone dans la partie alkylique et de radicaux alcoxycarbonyles contenant de 1 à 6 atomes de carbone dans la partie alkylique en tant que substituants faiblement électronégatifs, de substituants électriquement neutres et/ou de substituants électropositifs.

8. Procédé selon l'une quelconque des revendications 1 à 4, dans lequel on transforme un composé répondant à la formule générale:

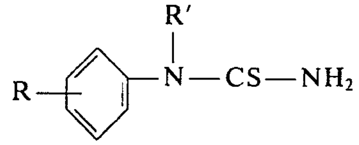

dans laquelle R' représente un atome d'hydrogène ou un alkyle éventuellement porteur d'un aryle éventuellement substitué et R représente un atome d'hydrogène, de fluor, de chlore ou de brome ou un radical alkyle en $C_1-C_6$, alcoxy en $C_1-C_6$, hydroxy ou amino, en un composé répondant à l'une des formules générales:

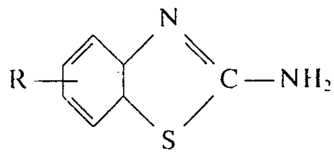 et 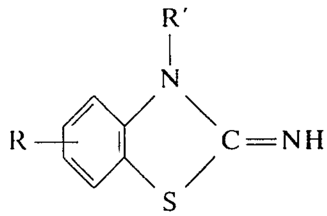

dans lesquelles R' et R ont les significations précédemment données.

9. Procédé selon l'une quelconque des revendications 1 à 4, dans lequel on transforme un comosé répondant à la formule:

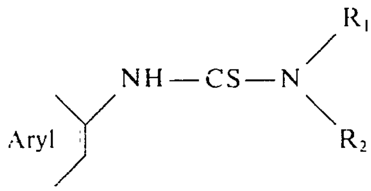

dans laquelle $R_1$ et $R_2$ sont identiques ou différents, $R_1$ représente un atome d'hydrogène, un alkyle en $C_1-C_8$ éventuellement porteur d'un halogène, d'un alcoxy en $C_1-C_6$ ou d'un phényle, ce dernier pouvant porter de 1 à 3 substituants choisis parmi les atomes d'halogène, les radicaux alcanoyles contenant de 1 à 6 atomes de carbone dans la partie alkylique et de radicaux alcoxycarbonyles contenant de 1 à 6 atomes de carbone dans la partie alkylique, comme substituants faiblement électronégatifs, électriquement neutres et/ou électropositifs, ou représente un cycloalkyle, $R_2$ représente un alkyle en $C_1-C_8$ qui peut porter un halogène, un alcoxy inférieur ou un phényle, ce dernier pouvant porter de 1 à 3 substituants pris dans l'ensemble constitué par les atomes d'halogène, les alcanoyles inférieurs et les alcoxycarbonyles inférieurs, comme substituants faiblement électronégatifs, électriquement neutres et/ou électropositifs, ou représente un cycloalkyle éventuellement substitué ou un aryle éventuellement substitué, et »Aryl« a la signification donnée à la revendication 5, en un composé répondant à la formule:

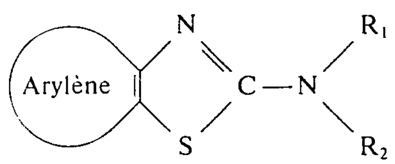

dans laquelle $R_1$ et $R_2$ réprésente ont les significations indiquées ci-dessus et »Arylène« a la signification donnée à la revendication 5.

10. Procédé selon l'une quelconque des revendications 1 à 4, dans lequel on transforme un composé répondant à la formule:

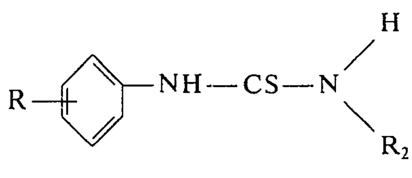

dans laquelle $R_2$ représente un alkyle en $C_1-C_6$, un cycloalkyle ou un aryle éventuellement substitué et R représente un atome d'hydrogène, de fluor, de chlore ou de brome, un alkyle en $C_1-C_6$, un alcoxy en $C_1-C_6$, un hydroxy ou un amino, en un composé répondant à la formule:

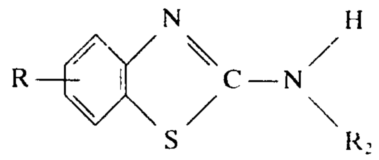

dans laquelle R et $R_2$ ont les significations précédemment données.